(19)

Europäisches Patentamt
European Patent Office
Office européen des brevets

(11) **EP 3 299 047 B1**

(12) **EUROPÄISCHE PATENTSCHRIFT**

(45) Veröffentlichungstag und Bekanntmachung des Hinweises auf die Patenterteilung:
**14.08.2019 Patentblatt 2019/33**

(21) Anmeldenummer: **17188239.2**

(22) Anmeldetag: **29.08.2017**

(51) Int Cl.:
*A61M 1/16* (2006.01)          *B01F 5/02* (2006.01)
*B01F 15/00* (2006.01)         *B01F 3/08* (2006.01)
*G05D 11/13* (2006.01)         *G05D 21/02* (2006.01)

(54) **VERFAHREN UND VORRICHTUNG ZUM VORMISCHEN VON DIALYSIERFLÜSSIGKEIT**

METHOD AND DEVICE FOR PRE-MIXING DIALYSATE

PROCÉDÉ ET DISPOSITIF DE PRÉMÉLANGE D'UN LIQUIDE DE DIALYSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.09.2016 DE 102016118172**

(43) Veröffentlichungstag der Anmeldung:
**28.03.2018 Patentblatt 2018/13**

(73) Patentinhaber: **B. Braun Avitum AG**
**34212 Melsungen (DE)**

(72) Erfinder:
• **GOLASZEWSKI, Grzegorz**
**34235 Lohfelden (DE)**
• **WAGNER, André**
**34117 Kassel (DE)**

(74) Vertreter: **Winter, Brandl, Fürniss, Hübner, Röss, Kaiser, Polte - Partnerschaft mbB Patent- und Rechtsanwaltskanzlei Alois-Steinecker-Strasse 22 85354 Freising (DE)**

(56) Entgegenhaltungen:
EP-A1- 1 491 222          EP-A2- 2 281 626
DE-A1- 10 214 699         DE-A1-102014 015 858
US-A1- 2014 107 835       US-B2- 6 991 190

**EP 3 299 047 B1**

**EP 3 299 047 B1**

**Beschreibung**

Die Erfindung betrifft ein Verfahren und eine Vorrichtung zum vorab erfolgenden Mischen von Dialysierflüssigkeit, und bezieht sich insbesondere auf ein Verfahren und eine Vorrichtung zum Vormischen von Dialysierflüssigkeit in einer Vorrichtung zur extrakorporalen Blutbehandlung mit anschließender Leitfähigkeitsmessung der vorgemischten Dialysierflüssigkeit.

Bei einer extrakorporalen Blutbehandlung, beispielsweise einer Dialyse, erfolgt aufgrund eines Konzentrationsgefälles zwischen Blut und Dialysierflüssigkeit ein diffusiver Transportvorgang zwischen dem Blut und der Dialysierflüssigkeit.

Dieser diffusive Transportvorgang ist im Wesentlichen für kleinmolekulare Urämietoxine, die in Abhängigkeit von ihrer Konzentration im Blut in die toxinfreie Dialysierflüssigkeit diffundieren, und für Elektrolyte und Puffersubstanzen, die entweder aus dem Blut entfernt (Kalium, Phosphat) oder dem Blut hinzugefügt werden müssen (Kalzium, Puffer), von Bedeutung. Die Höhe der Konzentration der Elektrolyte und Puffer in der Dialysierflüssigkeit bestimmt die Geschwindigkeit der Diffusion durch die Dialysemembran. Durch die Wahl der Dialysierflüssigkeitszusammensetzung lässt sich der Diffusionsprozess beeinflussen und anpassen.

Dialysierflüssigkeit wird in großer Menge benötigt. Bei einem Dialysierflüssigkeitsfluss von z.B. 500 ml/min beträgt die Dialysierflüssigkeitsmenge bei einer vier- bis fünfstündigen Dialysebehandlung ca. 120 - 150 Liter. Die Dialysierflüssigkeit wird durch Verdünnung von Konzentrat mit gereinigtem Osmosewasser produziert. Die Möglichkeit zur Variation der Dialysierflüssigkeitszusammensetzung durch gezielte Zumischung einzelner Bestandteile erlaubt eine Dialysebehandlung, die auf individuelle Bedürfnisse des Patienten zugeschnitten ist.

Für den Einsatz bei der Hämodialyse müssen Dialysierflüssigkeitslösungen bereitgestellt werden, die möglichst gut physiologisch angepasst sind, d.h. einen pH-Wert von etwa 7,4 aufweisen, wichtige Elektrolyten enthalten und ferner ein Puffersystem aufweisen, das physiologisch ist und sich zur Einstellung des gewünschten pH-Wertes eignet. Da Bicarbonat auch den physiologischen Puffer des Blutes darstellt, wird als Puffersystem im allgemeinen Bicarbonat verwendet.

Bei einer Vorrichtung zur extrakorporalen Blutbehandlung, beispielsweise einer Dialysemaschine, dient ein so genannter Dialysierflüssigkeitsblock (DF-Block) als Komponenten- oder Bauteileträger für unter anderem Leitfähigkeitssonden und Temperatursensoren der Dialysierflüssigkeitsaufbereitung. Hauptkomponenten der Dialysierflüssigkeitsaufbereitung sind in der Regel zumindest eine Bicarbonatpumpe und zumindest eine Konzentratpumpe, mit verschiedenen Leitfähigkeitsmesszellen, sowie zumindest eine Flusspumpe. Über die Bicarbonatpumpe zugemischtes Bicarbonatkonzentrat wird in einer Mischkammer zusammengeführt und mit einer Leitfähigkeitsmesszelle gemessen. Die Konzentrat- bzw. Säurekonzentratmischung erfolgt nach dem gleichen Prinzip und wird mit einer weiteren Leitfähigkeitsmesszelle gemessen.

Temperatursensoren sind für eine Temperaturkompensation der Leitfähigkeitsmessung verantwortlich. Die Temperaturerfassung eines ersten Temperatursensors wird nach der Zugabe kalten Konzentrats (zweite Messwertaufnahme für das Temperatursystem) durchgeführt, und die Temperaturerfassung eines zweiten Temperatursensors (und eines dritten Temperatursensors) wird unmittelbar vor dem Dialysator durchgeführt und dient damit zur Kompensation von Temperaturverlusten.

Die Leitfähigkeitsmesszelle oder -sonde ist eine unabhängig arbeitende Überwachungseinheit. Die Temperaturkompensation erfolgt über einen weiteren Temperatursensor.

Falls eine Proportionierung in einer Vorrichtung zur extrakorporalen Blutbehandlung, wie beispielsweise einer Dialysemaschine, mittels Rückschlagventilen und Mischkammern erfolgt, um eine Auswertung von Messsignalen der Leitfähigkeitssonden verwenden zu können, müssen, da die Messung der Leitfähigkeit unmittelbar hinter dem Hinzumischen der Konzentrate erfolgt, Permeat und Konzentrate aufwendig in Mischkammern durchmischt werden, um ein auswertbares Signal mit einem bisher verwendeten Tiefpass-Filteralgorithmus zu erhalten.

Darüber hinaus nimmt eine bekannte Lösung zur Messung des Leitfähigkeitssignals in der Vorrichtung einen hohen Flächen- und/oder Raumbedarf in Anspruch, und ist die Herstellung der Mischkammern und der Rückschlagventile mit hohen Produktions- und Montagkosten verbunden.

Beispielsweise ist in EP 1 491 222 A1 eine Dialysemaschine offenbart, bei welcher Leitungen für Dialysekonzentrate und osmotisches Wasser an einem Mischpunkt zusammengeleitet werden.

Ferner ist aus DE 10 2014 015 858 A1 eine Dialysevorrichtung bekannt, bei welcher zunächst pulverförmige Dialysekonzentrate und Wasser angemischt werden und diese anschließend in einer Mischkammer mit Wasser verdünnt werden.

US 2014/0107835 A1 offenbart ein allgemeines System zum Verteilen von Produkten, welches auch ein Mischen von Dialysekonzentrat in einem Krümmer als beispielhafte Anwendung beschreibt.

Der Erfindung liegt daher als eine Aufgabe zugrunde, eine Einrichtung zur vorab erfolgenden Mischung von Konzentraten und/oder Osmosewasser für eine extrakorporale Blutbehandlung zu schaffen, die einerseits ein Ausgasen der Konzentrate verhindert und dazu angeordnet ist, die Konzentrate zugunsten der Erreichung eines geforderten Durchmischungsgrads am Dialysator vorzumischen. Darüber hinaus soll ein robuster Filteralgorithmus bereitgestellt werden, mit welchem ein aus einem vorgemischten Konzentrat abgeleitetes Leitfähigkeitsmesssignal auswertbar ist.

Erfindungsgemäß wird diese Aufgabe durch eine Vorrichtung mit den Merkmalen des Anspruchs 1 und durch ein Verfahren mit den Merkmalen des Anspruchs 7 gelöst.

Vorteilhafte Weiterbildungen der Erfindung sind Gegenstand der beigefügten Unteransprüche.

Der Erfindung liegt als eine allgemeine Idee zugrunde, ein Ausgasen von Konzentraten zu verhindern und Konzentrate und Osmosewasser vorzumischen, und sodann die Leitfähigkeit der vorgemischten Dialysierflüssigkeit mittels eines digitalen Filters zu messen. Da der Durchmischungsgrad der Dialysierflüssigkeit bei der Zuführung zu den Leitfähigkeitssonden noch unzureichend ist und mit den derzeit bekannten Filteralgorithmen nicht gemessen werden kann, wird ferner ein robusterer Filteralgorithmus bereitgestellt um das Leitfähigkeitsmesssignal auswerten zu können.

Gemäß der allgemeinen Idee nutzt die Erfindung einen durch die Wassersäule erzeugten konstanten Vordruck, bevor die Konzentrate zugeführt werden, der eine Ausgasung der Konzentrate verhindert. Die Durchmischung der Konzentrate und des Osmosewassers erfolgt durch ein Gegenstromprinzip. Durch Messung der Leitfähigkeit am Dialysator wurde dabei festgestellt, dass der Durchmischungsgrad ohne Rückschlagventile und Mischkammern über die im Dialysierflüssigkeitssystem vorhandenen Komponenten ausreichend ist.

Um den Durchmischungsgrad am Dialysator sicher vorhersagen zu können und da die Regelung des Leitfähigkeitswerts auf digitale Art und Weise erfolgt, wird ein digitaler Filter eingesetzt, der insbesondere den aus dem dynamischen Charakter der Mischvorgänge resultierenden Anforderungen genügt.

Da die Durchmischung von Permeat und Konzentraten in kürzester Zeit erfolgen muss, erfolgt die Durchmischung im Gegenstromprinzip, um den erforderlichen Durchmischungsgrad an der Dialysatorkupplung zu erreichen. Die Durchmischung erfolgt innerhalb kürzester Zeit und mit (gesteuert) geringem Volumen der zu vermischenden Flüssigkeiten. Sie erfolgt außerdem turbulent mit daraus resultierenden Verwirbelungen, die zu einer guten Durchmischung führen.

Erfindungsgemäß ergeben sich Vorteile dahingehend, dass keine Rückschlagventile zur Verhinderung der Entgasung der Konzentrate erforderlich sind, keine Mischkammer zur Proportionierung erforderlich ist, der durch den Dialysierflüssigkeitsblock belegte Flächen- und/oder Raumbedarf eingespart werden kann, durch den Wegfall des Dialysierflüssigkeitsblocks Montagevorgänge vereinfacht sind, Heizspiegelschweißprozesse zur Herstellung des kostenintensiven Dialysierflüssigkeitsblocks entfallen, ein zu verarbeitendes Flüssigkeitsvolumen signifikant verringert wird, aufgrund der turbulenten Durchmischung im Gegenstromprinzip kurze Vermischungszeiten der Konzentrate und des Permeats erzielt werden, und der grundlegende Aufbau der Vorrichtung zur extrakorporalen Blutbehandlung bzw. Dialysemaschine erhalten bleibt. Außerdem vorteilhaft sind eine einfach anzuwendende Filtermethode für stark turbulente Signalformen, geringe Verzögerungszeiten und geringer Rechenaufwand aufgrund des einfachen Algorithmus, die Verzichtbarkeit eines Zustandsmodells des relevanten Systems und einer Kovarianzmatrix, eine einfache Implementierung und Parametrisierung und die Darstellbarkeit einer stabilen und zuverlässigen Leitfähigkeitsregelung.

Im Einzelnen wird die Aufgabe gelöst durch eine Vorrichtung zum Vormischen von Fluiden zu Dialysierflüssigkeit für eine extrakorporale Blutbehandlung, beinhaltend zumindest einen ersten Zulaufabschnitt zum Zuleiten zumindest eines ersten Fluids aus zumindest einem ersten Fluidvorrat; zumindest einen zweiten Zulaufabschnitt zum Zuleiten zumindest eines zweiten Fluids aus zumindest einem zweiten Fluidvorrat; einen Vormischabschnitt, der dazu konfiguriert ist, über die Zulaufabschnitte zugeleitete Fluide im Gegenstrom zu einem Fluidgemisch vorzumischen; und zumindest einen Ausleitabschnitt zum Ausleiten des in dem Vormischabschnitt vorgemischten Fluidgemischs als die Dialysierflüssigkeit. Es ist zumindest eine Leitfähigkeitsmesseinrichtung entlang des Ausleitabschnitts dazu vorgesehen, die Leitfähigkeit des vorgemischten Fluidgemischs zu messen und ein weiterverarbeitbares Leitfähigkeitsmesssignal zu erzeugen. Die zumindest eine Leitfähigkeitsmesseinrichtung umfasst ein digitales Filter, das auf der Grundlage zumindest zweier Zustandsgrößen des Vormischprozesses einstellbar ist, wobei eine der Zustandsgrößen von der jeweils anderen ableitbar ist und das digitale Filter zumindest zweiphasig mit einer ersten, prädiktiven Phase, in der ein nächster Systemzustand anhand von gemessenen Werten ermittelt wird, und einer zweiten, korrektiven Phase, in der die vorangehende Ermittlung anhand von Vergangenheitswerten korrigiert wird, konfiguriert ist.

Bevorzugt ist das erste Fluid Osmosewasser und ist das zweite Fluid ein Konzentrat einer für die extrakorporale Blutbehandlung zu verwendenden Substanz, weiter bevorzugt ein Bicarbonatkonzentrat.

Bevorzugt ist in dem zumindest einen Ausleitabschnitt eine Fluidsäule zur Erzeugung eines konstanten Vordrucks vorgesehen, die dazu konfiguriert ist, ein Ausgasen zumindest eines der zugeleiteten Fluide zu verhindern.

Bevorzugt sind der zumindest eine erste Zulaufabschnitt und der zumindest eine zweite Zulaufabschnitt bezüglich der Vorrichtung außenseitig und der Ausleitabschnitt zwischen dem zumindest einen ersten Zulaufabschnitt und dem zumindest einen zweiten Zulaufabschnitt angeordnet und derart fluidführend mit dem Vormischabschnitt verbunden, dass das zumindest eine erste Fluid aus einer ersten Richtung in den Vormischabschnitt einströmt und das zumindest eine zweite Fluid aus einer zweiten Richtung in den Vormischabschnitt einströmt, und nehmen der zumindest eine erste Zulaufabschnitt und der zumindest eine zweite Zulaufabschnitt einen vorbestimmten Winkel zueinander ein, der so konfiguriert ist, dass die gegenstromige Vormischung der Fluide in dem Vormischabschnitt strömungsbasiert turbulent und selbsttätig erzeugt wird.

Bevorzugt bilden die zwei Zustandsgrößen einen Zustandsvektor

$$v = \frac{dy}{dt} = \dot{y} \tag{1}$$

worin y eine Position und v eine Geschwindigkeit repräsentieren, und sind die erste und die zweite Phase des Filters durch die Gleichungen:

$$\hat{y}_{t+1} = \hat{y}_t + \Delta T \cdot \hat{v}_t \tag{2}$$

$$\hat{v}_{t+1} = \hat{v}_t \tag{3}$$

$$\hat{y}_t = \hat{y}_{t-1} + \alpha \cdot (y_t - \hat{y}_{t-1}) \tag{4}$$

$$\hat{v}_t = \hat{v}_{t-1} + \frac{\beta}{\Delta T} \cdot (y_t - \hat{y}_{t-1}) \tag{5}$$

bestimmt, worin die Gleichungen (2) und (3) die prädiktive Phase und die Gleichungen (4) und (5) die korrektive Phase repräsentieren, die Größen in den Gleichungen Schätzwerte der jeweils gemessenen Größe und die Abtastzeit zwischen zwei aufeinander folgenden Messungen repräsentieren, der Index t den aktuellen Wert in Bezug auf die aktuelle Messung angibt, der Index t-1 den Wert in Bezug auf die jeweils letzte Messung angibt, der Index t+1 den Wert in Bezug auf die jeweils nächste Messung angibt, und Filtereigenschaften über einen Parameter $\alpha$ und einen Parameter $\beta$ einstellbar sind.

Im Einzelnen wird die Aufgabe außerdem gelöst durch ein Verfahren zum Vormischen von Fluiden zu Dialysierflüssigkeit für eine extrakorporale Blutbehandlung, beinhaltend die Schritte: Zuleiten zumindest eines ersten Fluids aus zumindest einem ersten Fluidvorrat über zumindest einen ersten Zulaufabschnitt; Zuleiten zumindest eines zweiten Fluids aus zumindest einem zweiten Fluidvorrat über zumindest einen zweiten Zulaufabschnitt; Vormischen der über die Zulaufabschnitte zugeleiteten Fluide im Gegenstrom zu einem Fluidgemisch in einem Vormischabschnitt; und Ausleiten des in dem Vormischabschnitt vorgemischten Fluidgemischs als Dialysierflüssigkeit über zumindest einen Ausleitabschnitt. Ferner beinhaltet das Verfahren ferner ein Durchführen einer Leitfähigkeitsmessung entlang des Ausleitabschnitts zur Messung der Leitfähigkeit des vorgemischten Fluidgemischs zum Erzeugen eines weiterverarbeitbaren Leitfähigkeitsmesssignals, wobei bei der Leitfähigkeitsmessung die Leitfähigkeit mittels eines parametrierbaren digitalen Filters zweiphasig, mit einer ersten, prädiktiven Phase und einer zweiten, korrektiven Phase, auf der Grundlage zweier Zustandsgrößen des Vormischprozesses gemessen wird. In anderen Worten wird die Leitfähigkeitsmessung mittels eines digitalen Filters durchgeführt, das auf der Grundlage zumindest zweier Zustandsgrößen des Vormischprozesses einstellbar ist, wobei vorzugsweise eine der Zustandsgrößen von der jeweils anderen ableitbar ist und das digitale Filter zumindest zweiphasig mit einer ersten, prädiktiven Phase, in der vorzugsweise ein nächster Systemzustand anhand von gemessenen Werten ermittelt wird, und einer zweiten, korrektiven Phase, in der vorzugsweise die vorangehende Ermittlung anhand von Vergangenheitswerten korrigiert wird, konfiguriert ist. Vorzugsweise bilden die zwei Zustandsgrößen einen Zustandsvektor

$$v = \frac{dy}{dt} = \dot{y} \tag{1}$$

, worin y eine Position und v eine Geschwindigkeit repräsentieren, und die erste und die zweite Phase des Filters durch die Gleichungen:

$$\hat{y}_{t+1} = \hat{y}_t + \Delta T \cdot \hat{v}_t \tag{2}$$

$$\hat{v}_{t+1} = \hat{v}_t \qquad\qquad (3)$$

$$\hat{y}_t = \hat{y}_{t-1} + \alpha \cdot (y_t - \hat{y}_{t-1}) \qquad\qquad (4)$$

$$\hat{v}_t = \hat{v}_{t-1} + \frac{\beta}{\Delta T} \cdot (y_t - \hat{y}_{t-1}) \qquad\qquad (5)$$

bestimmt sind, worin die Gleichungen (2) und (3) die prädiktive Phase und die Gleichungen (4) und (5) die korrektive Phase repräsentieren, die Größen in den Gleichungen Schätzwerte der jeweils gemessenen Größe und die Abtastzeit zwischen zwei aufeinander folgenden Messungen repräsentieren, der Index t den aktuellen Wert in Bezug auf die aktuelle Messung angibt, der Index t-1 den Wert in Bezug auf die jeweils letzte Messung angibt, der Index t+1 den Wert in Bezug auf die jeweils nächste Messung angibt, und Filtereigenschaften über einen Parameter $\alpha$ und einen Parameter $\beta$ einstellbar sind.

Die Erfindung wird nachstehend anhand bevorzugter Ausführungsbeispiele unter Bezugnahme auf die Zeichnung näher beschrieben. Es zeigen:

Fig. 1 vereinfacht eine Vorrichtung zum vorab erfolgenden Mischen von Dialysierflüssigkeit als eine in einer Vorrichtung zur extrakorporalen Blutbehandlung verbaubare Komponente gemäß einem ersten Ausführungsbeispiel;

Fig. 2 schematisch eine Prinzipdarstellung der Vormischung von Dialysierflüssigkeit in der Vorrichtung zum vorab erfolgenden Mischen von Dialysierflüssigkeit gemäß dem ersten Ausführungsbeispiel;

Fig. 3 ein Diagramm eines Verlaufs beispielhaft erhaltener Leitfähigkeitsmesssignale; und

Fig. 4 ein Diagramm von Leitfähigkeitsmesssignalen nach Verarbeitung unter Verwendung eines Filteralgorithmus gemäß einem zweiten Ausführungsbeispiel.

Es wird angemerkt, dass in der Zeichnung gleiche oder gleichwirkende Elemente und Komponenten mit denselben Bezugszeichen bezeichnet sind und nicht redundant beschrieben werden.

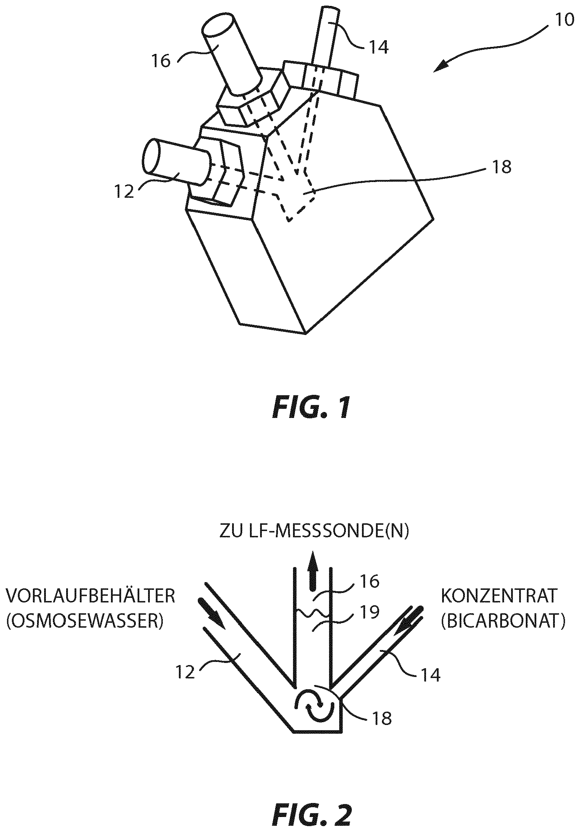

Fig. 1 zeigt vereinfacht eine Vormischvorrichtung 10 zum vorab erfolgenden Mischen von Dialysierflüssigkeit als eine in einer Vorrichtung zur extrakorporalen Blutbehandlung, wie beispielsweise einer Dialysemaschine, verbaubare Komponente gemäß einem ersten Ausführungsbeispiel. Das hier zugrundeliegende Konzept der Vormischung hat zwei Aufgaben zu erfüllen, zum einen ein Vormischen der Konzentrate mit dem Osmosewasser und zum anderen ein Verhindern des Ausgasens der Konzentrate, insbesondere von Bicarbonat.

Gemäß dem vorliegenden Ausführungsbeispiel weist die Vormischvorrichtung 10 einen ersten Zulauf 12 zur (maschinenseitig mengengesteuerten) Zuführung des in einem Vorlaufbehälter (nicht gezeigt) erwärmten Osmosewassers, einen zweiten Zulauf 14 zur (maschinenseitig mengengesteuerten) Zuführung zumindest eines Konzentrats direkt von einer Drehschieberkolbenpumpe (nicht gezeigt) der Vorrichtung zur extrakorporalen Blutbehandlung und einen Auslauf 16 zur Ausleitung der vorgemischten Dialysierflüssigkeit aus der Vormischvorrichtung 10 und Vorbeiführung oder Zuführung der vorgemischten Dialysierflüssigkeit an oder zu zumindest einer Leitfähigkeitssonde (nicht gezeigt) auf.

In einem Vormischabschnitt 18 der Vormischvorrichtung 10 erfolgt eine Vermischung oder Vormischung des Osmosewassers als einem ersten Fluid mit dem zumindest einen Konzentrat als einem zweiten Fluid im Gegenstromprinzip, d.h. turbulent unter Verwirbelung des aus einer ersten Richtung über den ersten Zulauf 12 in den Vormischabschnitt 18 einströmenden Osmosewassers und des aus einer zweiten Richtung über den zweiten Zulauf 14 einströmenden zumindest einen Konzentrats.

Eine über dem Vormischabschnitt 18 in dem Auslauf 16 stehende Wassersäule 19 verhindert dabei ein Ausgasen der Konzentrate, insbesondere des Bicarbonats.

Aus dem Auslauf 16 ausgeleitete, vorgemischte Dialysierflüssigkeit wird zumindest einer Leitfähigkeitsmesszelle oder -sonde (nicht gezeigt) zugeleitet, welche die vorgemischte Dialysierflüssigkeit bezüglich ihrer Leitfähigkeit vermisst und ein weiterverarbeitbares Leitfähigkeitsmesssignal erzeugt.

Es versteht sich, dass die vorstehend beschriebene Vormischvorrichtung 10 nicht auf die in dem ersten Ausführungsbeispiel beispielsweise dargestellte Form oder Anzahl von Anschlüssen bzw. Einlässen und Auslässen beschränkt ist. Ferner können in einer Vorrichtung zur extrakorporalen Blutbehandlung eine oder mehrere Vormischvorrichtung(en) 10 vorgesehen und angeordnet sein. Eine vorzugsweise Anordnung der Vormischvorrichtung(en) 10 kann beispielsweise maschinenintern auf einem Träger in einem die Herstellung der erforderlichen Fluidverbindungen erlaubenden Abstand zu Fluidvorräten und/oder Pumpeinrichtungen vorgesehen sein.

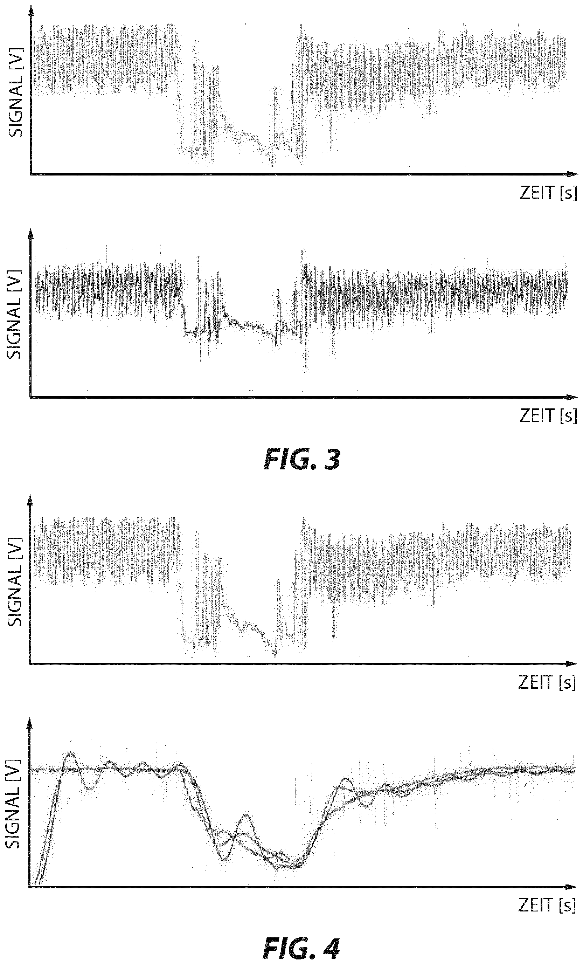

Fig. 3 zeigt ein Diagramm eines Verlaufs von über eine Zeitdauer T (in beispielsweise Sekunden) beispielhaft erhaltener Leitfähigkeitsmesssignale (einer Spannung in beispielsweise Volt). Wie dem oberen Teil der Fig. 3 zu entnehmen ist, ist das ursprünglich erfasste Leitfähigkeitsmesssignal signifikant verrauscht. Es kann mit bisher bekannten und eingesetzten verwendeten Filtermethoden nicht für eine stabile Regelung verwendet werden.

Zunächst können daher die Messgrößen mittels beispielsweise Mittelwertbildung oder gleitender Mittelwertbildung von in geringerem Maße einwirkenden Störeinflüssen befreit werden. Ein beispielsweise resultierendes gefiltertes Leitfähigkeitssignal ist im unteren Teil der Fig. 3 dargestellt.

Soll die Filterwirkung stärker sein, kann der Grad des Filters erhöht werden. Aufwändigere Filterungsarten, wie z.B. ein FIR-Filter oder ein IIR-Filter, besitzen zwar bei höherem Grad eine bessere Filterwirkung, bringen aber eine deutliche Verzögerung für das Gesamtsystem mit sich. Die Verzögerung ist zu dem Grad des Filters direkt proportional und wächst somit ebenso proportional mit der Filterwirkung. Zusätzlich ergeben Filterarten wie beispielsweise ein IIR-Filter keine stationäre Impulsantwort, so dass eine Anwendung in manchen Bereichen erschwert oder sogar unmöglich ist.

Zwar werden bei komplexen Filteralgorithmen, wie z.B. einem Kaiman-Filter, die Ergebnisse gut gefiltert. Allerdings benötigt diese Filterung eine genauere Systembeschreibung in Form eines Zustandsmodells. Ein Zustandsmodell wiederum ist bei komplexeren Systemen wie beispielsweise einer Dialysemaschine nicht praktikabel.

Zusätzlich werden dabei aufwändige Rechenoperationen mit Matrizen notwendig, die die erforderliche Rechenleistung wesentlich erhöhen und die Parametrisierung des Filters komplexer gestalten.

Eine Alternative zu den oben genannten Filtermethoden stellt gemäß einem zweiten Ausführungsbeispiel ein aufgrund verwendeter (bzw. benannter) Parameter $\alpha$, $\beta$ so bezeichnetes digitales Alpha-Beta-Filter dar. Bei einem Alpha-Beta-Filter in diesem Ausführungsbeispiel handelt es sich um einen prädiktiven Algorithmus, welcher einen vereinfachten Beobachter für eine Prognose darstellt. Der Alpha-Beta-Filter basiert auf einer Annahme dahingehend, dass ein Prozess durch zwei Zustandsgrößen eindeutig bestimmbar ist, wobei eine der Zustandsgrößen von der jeweils anderen abgeleitet werden kann.

Diese Annahme wird gemäß dem vorliegenden Ausführungsbeispiel durch einen Zustandsvektor, der sich aus der Position (y) und der Geschwindigkeit (v) zusammensetzt, erfüllt:

$$v = \frac{dy}{dt} = \dot{y} \qquad\qquad (1)$$

Der Filteralgorithmus setzt sich sodann aus zwei Phasen zusammen, einer ersten, prädiktiven Phase und einer zweiten, korrektiven Phase. In der ersten, prädiktiven Phase wird der nächste Systemzustand anhand von gemessenen Werten ermittelt. In der zweiten, korrektiven Phase wird die vorangehende Ermittlung anhand von Vergangenheitswerten korrigiert.

Die beiden vorgenannten Phasen des Algorithmus können in den nachstehenden Gleichungen zusammengefasst werden:

$$\hat{y}_{t+1} = \hat{y}_t + \Delta T \cdot \hat{v}_t \qquad\qquad (2)$$

$$\hat{v}_{t+1} = \hat{v}_t \qquad\qquad (3)$$

$$\hat{y}_t = \hat{y}_{t-1} + \alpha \cdot (y_t - \hat{y}_{t-1}) \qquad\qquad (4)$$

$$\hat{v}_t = \hat{v}_{t-1} + \frac{\beta}{\Delta T} \cdot (y_t - \hat{y}_{t-1}) \qquad\qquad (5)$$

worin die Gleichungen (2) und (3) die prädiktive Phase und die Gleichungen (4) und (5) die korrektive Phase repräsentieren.

Die Größen in den Gleichungen bezeichnen geschätzte Werte der gemessenen Größe und der Abtastzeit zwischen zwei aufeinander bzw. nacheinander folgenden Messungen. Der Index t gibt den aktuellen Wert in Bezug auf die aktuelle Messung, der Index t-1 den Wert in Bezug auf die letzte Messung und der Index t+1 den Wert in Bezug auf die nächste Messung an. Die beiden Parameter $\alpha$ und $\beta$ werden zur Einstellung der Filtereigenschaften verwendet. Diese Parameter $\alpha$ und $\beta$ sollten möglichst im Bereich von 0 bis 1 gewählt werden, um Störungen zu unterdrücken.

Wie vorstehend beschrieben wurde, basiert das vorgenannte Alpha-Beta-Filter auf der Annahme, dass ein Prozess durch zwei Zustandsgrößen eindeutig bestimmbar ist, und entspricht bei dem Filter Alpha dem Weg und Beta der Geschwindigkeit. Die Erfindung ist jedoch nicht auf ein solches Alpha-Beta-Filter mit zwei Zustandsgrößen bzw. Phasen beschränkt.

Das Alpha-Beta-Filter kann dem Grunde nach beliebig und beispielsweise um eine weitere Gleichung erweitert werden, die die Beschleunigung darstellt. Wird die Beschleunigung mit zum Beispiel Gamma bezeichnet, kann in der erweiterten Form ein Alpha-Beta-Gamma Filter dargestellt werden. Ein solches Alpha-Beta-Gamma Filter kann bei vertretbar größerem Rechenaufwand vorteilhaft eine nochmals verbesserte Filterwirkung bereitstellen.

Durch entsprechendes Hinzufügen weiterer Gleichungen und somit weiterer Phasen des Filters nach dem vorgenannten Prinzip können in Modifikationen Erweiterungen beliebig umfangreich werden, wobei die Verwendung oder Anwendbarkeit einer Erweiterung rein anwendungsfallabhängig sein kann. In anderen Worten hängt es dann fallweise nur noch von einem Anwendungsfall ab, ob eine Erweiterung in Betracht gezogen wird.

Fig. 4 zeigt ausgehend von dem ursprünglich erfassten Signalverlauf (oberer Teil der Fig. 4) ein Diagramm von Leitfähigkeitsmesssignalen nach Verarbeitung unter Verwendung des Filteralgorithmus gemäß dem zweiten Ausführungsbeispiel (unterer Teil der Fig. 4).

Die Ausgangsmesswerte (hier beispielsweise entsprechend dem oberen Teil der Fig. 3) wurden dazu mit Hilfe des vorstehend beschriebenen Filters bzw. Filteralgorithmus verarbeitet und sind im unteren Teil der Fig. 4 als die im linksseitigen Anfangsbereich nahezu konstant verlaufende und insgesamt glatteste der Messwertverlaufskurven dargestellt. Ferner sind im unteren Teil der Fig. 4 zur Verdeutlichung der Effektivität des Filters zwei vergleichsweise Kurven jeweils eines FIR-Tiefpassfilters und eines IIR-Tiefpassfilters mit jeweils Überschwingern und Unterschwingern um eine mit dem Filter gemäß dem vorliegenden Ausführungsbeispiel verarbeitete Messwertverlaufskurve dargestellt.

Wie vorstehend beschrieben wurde, beinhaltet eine Vorrichtung zum Vormischen von Fluiden zu Dialysierflüssigkeit für eine extrakorporale Blutbehandlung zumindest einen ersten Zulaufabschnitt zum Zuleiten zumindest eines ersten Fluids aus zumindest einem ersten Fluidvorrat, zumindest einen zweiten Zulaufabschnitt zum Zuleiten zumindest eines zweiten Fluids aus zumindest einem zweiten Fluidvorrat, einen Vormischabschnitt, der dazu konfiguriert ist, über die Zulaufabschnitte zugeleitete Fluide im Gegenstrom zu einem Fluidgemisch vorzumischen, und zumindest einen Ausleitabschnitt zum Ausleiten des in dem Vormischabschnitt vorgemischten Fluidgemischs als die Dialysierflüssigkeit, und führt ein Verfahren entsprechende Schritte aus. In einer Leitfähigkeitsmessung entlang des Ausleitabschnitts wird die Leitfähigkeit des vorgemischten Fluidgemischs mittels eines parametrierbaren digitalen Filters auf der Grundlage zweier Zustandsgrößen des Vormischprozesses zweiphasig mit einer ersten, prädiktiven Phase und einer zweiten, korrektiven Phase gemessen.

Die Erfindung wurde vorstehend anhand bevorzugter Ausführungsbeispiele beschrieben. Es versteht sich, dass Einzelheiten der beschriebenen bevorzugten Ausführungsbeispiele die Erfindung als solche nicht beschränken und sich für den Fachmann naheliegend verschiedenartige Änderungen, Modifikationen und/oder Äquivalente ergeben können, die als solche allesamt im Rahmen der durch die beigefügten Ansprüche definierten Schutzbereich der Erfindung liegen.

**Patentansprüche**

1. Vorrichtung zum Vormischen von Fluiden zu Dialysierflüssigkeit für eine extrakorporale Blutbehandlung, mit:

zumindest einem ersten Zulaufabschnitt (12) zum Zuleiten zumindest eines ersten Fluids aus zumindest einem ersten Fluidvorrat;
zumindest einem zweiten Zulaufabschnitt (14) zum Zuleiten zumindest eines zweiten Fluids aus zumindest einem zweiten Fluidvorrat;
einem Vormischabschnitt (18), der dazu konfiguriert ist, über die Zulaufabschnitte (12, 14) zugeleitete Fluide im Gegenstrom zu einem Fluidgemisch vorzumischen; und
zumindest einem Ausleitabschnitt (16) zum Ausleiten des in dem Vormischabschnitt (18) vorgemischten Fluidgemischs als die Dialysierflüssigkeit, wobei
zumindest eine Leitfähigkeitsmesseinrichtung entlang des Ausleitabschnitts (16) dazu vorgesehen ist, die Leitfähigkeit des vorgemischten Fluidgemischs zu messen und ein weiterverarbeitbares Leitfähigkeitsmesssignal zu erzeugen,
**dadurch gekennzeichnet, dass**
die Leitfähigkeitsmesseinrichtung ein digitales Filter umfasst, das auf der Grundlage zumindest zweier Zustandsgrößen des Vormischprozesses einstellbar ist, wobei eine der Zustandsgrößen von der jeweils anderen ableitbar ist und das digitale Filter zumindest zweiphasig mit einer ersten, prädiktiven Phase, in der ein nächster Systemzustand anhand von gemessenen Werten ermittelt wird, und einer zweiten, korrektiven Phase, in der die vorangehende Ermittlung anhand von Vergangenheitswerten korrigiert wird, konfiguriert ist.

2. Vorrichtung nach Anspruch 1, bei der das erste Fluid Osmosewasser ist und das zweite Fluid ein Konzentrat einer für die extrakorporale Blutbehandlung zu verwendenden Substanz ist.

3. Vorrichtung nach Anspruch 2, bei der das Konzentrat ein Bicarbonatkonzentrat ist.

4. Vorrichtung nach einem der vorangehenden Ansprüche, bei der in dem zumindest einen Ausleitabschnitt (16) eine Fluidsäule (19) zur Erzeugung eines konstanten Vordrucks vorgesehen ist, die dazu konfiguriert ist, ein Ausgasen zumindest eines der zugeleiteten Fluide zu verhindern.

5. Vorrichtung nach einem der vorangehenden Ansprüche, bei der der zumindest eine erste Zulaufabschnitt (12) und der zumindest eine zweite Zulaufabschnitt (14) bezüglich der Vorrichtung (10) außenseitig und der Ausleitabschnitt (16) zwischen dem zumindest einen ersten Zulaufabschnitt (12) und dem zumindest einen zweiten Zulaufabschnitt (14) angeordnet und derart fluidführend mit dem Vormischabschnitt (18) verbunden sind, dass das zumindest eine erste Fluid aus einer ersten Richtung in den Vormischabschnitt (18) einströmt und das zumindest eine zweite Fluid aus einer zweiten Richtung in den Vormischabschnitt (18) einströmt, und der zumindest eine erste Zulaufabschnitt (12) und der zumindest eine zweite Zulaufabschnitt (14) einen vorbestimmten Winkel zueinander einnehmen, der so konfiguriert ist, dass die gegenstromige Vormischung der Fluide in dem Vormischabschnitt (18) strömungsbasiert turbulent und selbsttätig erzeugt wird.

6. Vorrichtung nach einem der vorangehenden Ansprüche, bei der die zumindest zwei Zustandsgrößen einen Zustandsvektor

$$v = \frac{dy}{dt} = \dot{y} \tag{1}$$

bilden, worin y eine Position und v eine Geschwindigkeit repräsentieren, und die erste und die zweite Phase des Filters durch die Gleichungen:

$$\hat{y}_{t+1} = \hat{y}_t + \Delta T \cdot \hat{v}_t \tag{2}$$

$$\hat{v}_{t+1} = \hat{v}_t \tag{3}$$

$$\hat{y}_t = \hat{y}_{t-1} + \alpha \cdot (y_t - \hat{y}_{t-1}) \tag{4}$$

$$\hat{v}_t = \hat{v}_{t-1} + \frac{\beta}{\Delta T} \cdot (y_t - \hat{y}_{t-1}) \tag{5}$$

bestimmt sind, worin die Gleichungen (2) und (3) die prädiktive Phase und die Gleichungen (4) und (5) die korrektive Phase repräsentieren, die Größen in den Gleichungen Schätzwerte der jeweils gemessenen Größe und die Abtastzeit zwischen zwei aufeinander folgenden Messungen repräsentieren, der Index t den aktuellen Wert in Bezug auf die aktuelle Messung angibt, der Index t-1 den Wert in Bezug auf die jeweils letzte Messung angibt, der Index t+1 den Wert in Bezug auf die jeweils nächste Messung angibt, und Filtereigenschaften über einen Parameter $\alpha$ und einen Parameter $\beta$ einstellbar sind.

7. Verfahren zum Vormischen von Fluiden zu Dialysierflüssigkeit für eine extrakorporale Blutbehandlung, mit den Schritten:

Zuleiten zumindest eines ersten Fluids aus zumindest einem ersten Fluidvorrat über zumindest einen ersten Zulaufabschnitt (12);

Zuleiten zumindest eines zweiten Fluids aus zumindest einem zweiten Fluidvorrat über zumindest einen zweiten Zulaufabschnitt (14);

Vormischen der über die Zulaufabschnitt zugeleiteten Fluide im Gegenstrom zu einem Fluidgemisch in einem Vormischabschnitt (18);

Ausleiten des in dem Vormischabschnitt vorgemischten Fluidgemischs als die Dialysierflüssigkeit über zumindest einen Ausleitabschnitt (16); und

Durchführen einer Leitfähigkeitsmessung entlang des Ausleitabschnitts (16) zur Messung der Leitfähigkeit des vorgemischten Fluidgemischs, **dadurch gekennzeichnet, dass**

bei der Leitfähigkeitsmessung die Leitfähigkeit mittels eines parametrierbaren digitalen Filters auf der Grundlage zweier Zustandsgrößen des Vormischprozesses zweiphasig mit einer ersten, prädiktiven Phase und einer zweiten, korrektiven Phase gemessen wird.

8. Verfahren nach Anspruch 7, wobei

eine der Zustandsgrößen von der jeweils anderen ableitbar ist,

in der ersten, prädiktiven Phase ein nächster Systemzustand anhand von gemessenen Werten ermittelt wird, und

in der zweiten, korrektiven Phase die vorangehende Ermittlung anhand von Vergangenheitswerten korrigiert wird, und

wobei die zwei Zustandsgrößen einen Zustandsvektor

$$v = \frac{dy}{dt} = \dot{y} \tag{1}$$

bilden, worin y eine Position und v eine Geschwindigkeit repräsentieren, und die erste und die zweite Phase des Filters durch die Gleichungen:

$$\hat{y}_{t+1} = \hat{y}_t + \Delta T \cdot \hat{v}_t \tag{2}$$

$$\hat{v}_{t+1} = \hat{v}_t \tag{3}$$

$$\hat{y}_t = \hat{y}_{t-1} + \alpha \cdot (y_t - \hat{y}_{t-1}) \tag{4}$$

$$\hat{v}_t = \hat{v}_{t-1} + \frac{\beta}{\Delta T} \cdot (y_t - \hat{y}_{t-1}) \tag{5}$$

bestimmt sind, worin die Gleichungen (2) und (3) die prädiktive Phase und die Gleichungen (4) und (5) die korrektive Phase repräsentieren, die Größen in den Gleichungen Schätzwerte der jeweils gemessenen Größe und die Abtastzeit zwischen zwei aufeinander folgenden Messungen repräsentieren, der Index t den aktuellen Wert in Bezug auf die aktuelle Messung angibt, der Index t-1 den Wert in Bezug auf die jeweils letzte Messung angibt, der Index t+1 den Wert in Bezug auf die jeweils nächste Messung angibt, und Filtereigenschaften über einen Parameter $\alpha$ und einen Parameter $\beta$ einstellbar sind.

## Claims

1. An apparatus for premixing fluids to form a dialysate for an extracorporeal blood treatment, comprising:

at least a first feed portion (12) for supplying at least a first fluid from at least a first fluid reservoir;

at least a second feed portion (14) for supplying at least a second fluid from at least a second fluid reservoir;

a premixing portion (18) which is configured to premix fluids supplied via the feed portions (12, 14) in counter flow to form a fluid mixture; and

at least one drain portion (16) for draining the fluid mixture premixed in the premixing portion (18) as the dialysate, wherein

at least one conductivity measuring device along the drain portion (16) is provided for measuring the conductivity of the premixed fluid mixture and to generate a conductivity measuring signal capable of being further processed, **characterized in that**

the conductivity measuring device comprises a digital filter which is adjustable on the basis of at least two state variables of the premixing process, wherein one of the state variables can be derived from the respective other one, and the digital filter is configured to have at least two phases, comprising a first, predictive phase in which a next system state is determined by way of measured values and a second, corrective phase in which the preceding finding is corrected by way of previous values.

2. The apparatus according to claim 1, wherein the first fluid is osmotic water and the second fluid is a concentrate of a substance to be used for the extracorporeal blood treatment.

3. The apparatus according to claim 2, wherein the concentrate is a bicarbonate concentrate.

4. The apparatus according to any one of the preceding claims, wherein in the at least one drain portion (16) a fluid column (19) for generating a constant primary pressure is provided which is configured to prevent at least one of the supplied fluids from outgassing.

5. The apparatus according to any one of the preceding claims, wherein the at least one first feed portion (12) and the at least one second feed portion (14) are arranged with respect to the apparatus (10) on the outside and the drain portion (16) is arranged between the at least one first feed portion (12) and the at least one second feed portion (14) and they are connected to the premixing portion (18) in a fluid-guiding manner so that the at least one first fluid flows from a first direction into the premixing portion (18) and the at least one second fluid flows from a second direction into the premixing portion (18), and the at least one first feed portion (12) and the at least one second feed portion (14) adopt a predetermined angle with each other which is configured so that the counter-flow premixing of the fluids is generated in the premixing portion (18) in a turbulent and automatic way based on flow.

6. The apparatus according to one of the preceding claims, wherein the at least two state variables form a state vector

$$v = \frac{dy}{dt} = \dot{y} \qquad (1)$$

wherein y represents a position and v represents a velocity and the first and second phases of the filter are determined by the equations:

$$\hat{y}_{t+1} = \hat{y}_t + \Delta T \cdot \hat{v}_t \qquad (2)$$

$$\hat{v}_{t+1} = \hat{v}_t \qquad (3)$$

$$\hat{y}_t = \hat{y}_{t-1} + \alpha \cdot (y_t - \hat{y}_{t-1}) \qquad (4)$$

$$\hat{v}_t = \hat{v}_{t-1} + \frac{\beta}{\Delta T} \cdot (y_t - \hat{y}_{t-1}) \qquad (5)$$

wherein the equations (2) and (3) represent the predictive phase and the equations (4) and (5) represent the corrective phase, the variables in the equations represent estimates of the respectively measured variable and the scanning time between two successive measurements, the index t indicates the current value with respect to the current measurement, the index t-1 indicates the value with respect to the respective last measurement, the index t+1 indicates the value with respect to the respective next measurement, and filter characteristics are adjustable via a parameter $\alpha$ and a parameter $\beta$.

7. A method for premixing fluids to form dialysate for an extracorporeal blood treatment, comprising the steps of:

   supplying at least a first fluid from at least a first fluid reservoir via at least a first feed portion (12);
   supplying at least a second fluid from at least a second fluid reservoir via at least a second feed portion (14);
   premixing the fluids supplied via the feed portions in counter flow to form a fluid mixture in a premixing portion (18); and
   draining the fluid mixture premixed in the premixing portion as the dialysate via at least one drain portion (16), and performing a conductivity measurement along the drain portion (16) for measuring the conductivity of the premixed fluid mixture
   **characterized in that**
   for the conductivity measurement, the conductivity is measured by means of a digital filter adapted to be parameterized based on two state variables of the premixing process in two phases with a first predictive phase and a second corrective phase.

8. The method according to claim 7, wherein one of the state variables can be derived from the respective other one, in the first, predictive phase a next system state is determined by way of measured values, and
   in the second, corrective phase the preceding finding is corrected by way of previous values, and
   wherein the two state variables form a state vector

$$v = \frac{dy}{dt} = \dot{y} \tag{1}$$

wherein y represents a position and v represents a velocity, and the first and second phases of the filter are determined by the equations:

$$\hat{y}_{t+1} = \hat{y}_t + \Delta T \cdot \hat{v}_t \tag{2}$$

$$\hat{v}_{t+1} = \hat{v}_t \tag{3}$$

$$\hat{y}_t = \hat{y}_{t-1} + \alpha \cdot (y_t - \hat{y}_{t-1}) \tag{4}$$

$$\hat{v}_t = \hat{v}_{t-1} + \frac{\beta}{\Delta T} \cdot (y_t - \hat{y}_{t-1}) \tag{5}$$

wherein the equations (2) and (3) represent the predictive phase and the equations (4) and (5) represent the corrective phase, the variables in the equations represent estimates of the respectively measured variable and the scanning time between two successive measurements, the index t indicates the current value with respect to the current measurement, the index t-1 indicates the value with respect to the respective last measurement, the index t+1 indicates the value with respect to the respective next measurement, and filter characteristics are adjustable via a parameter α and a parameter β.

**Revendications**

1. Dispositif de prémélange de fluides en liquide de dialyse pour un traitement du sang extracorporel, avec :

   au moins une première section d'amenée (12) pour l'amenée d'au moins un premier fluide à partir d'au moins un premier réservoir de fluide ;
   au moins une deuxième section d'amenée (14) pour l'amenée d'au moins un deuxième fluide à partir d'au moins un deuxième réservoir de fluide ;
   une section de prémélange (18), qui est configurée pour prémélanger des fluides amenés par le biais des sections d'amenée (12, 14) à contre-courant en un mélange de fluide ; et

au moins une section d'évacuation (16) pour l'évacuation du mélange de fluide prémélangé dans la section de prémélange (18) en tant que fluide de dialyse, dans lequel

au moins un dispositif de mesure de conductibilité est prévu le long de la section d'évacuation (16) pour mesurer la conductibilité du mélange de fluide prémélangé et pour générer un signal de mesure de conductibilité pouvant être traité,

**caractérisé en ce que**

le dispositif de mesure de conductibilité comprend un filtre numérique, qui est réglable sur la base d'au moins deux grandeurs d'état du processus de prémélange, dans lequel une des grandeurs d'état peut être déduite de respectivement l'autre et le filtre numérique est configuré au moins biphasé avec une première phase prédictive, dans laquelle un prochain état de système est déterminé à l'aide de valeurs mesurées, et une deuxième phase corrective, dans laquelle la détermination précédente est corrigée à l'aide de valeurs historiques.

2.  Dispositif selon la revendication 1, dans lequel le premier fluide est de l'eau osmosée et le deuxième fluide est un concentré d'une substance à utiliser pour le traitement du sang extracorporel.

3.  Dispositif selon la revendication 2, dans lequel le concentré est un concentré de bicarbonate.

4.  Dispositif selon l'une quelconque des revendications précédentes, dans lequel une colonne de fluide (19) est prévue dans l'au moins une section d'évacuation (16) pour la génération d'une pré-pression constante, qui est configurée pour empêcher un dégazage d'au moins un des fluides amenés.

5.  Dispositif selon l'une quelconque des revendications précédentes, dans lequel l'au moins une première section d'amenée (12) et l'au moins une deuxième section d'amenée (14) sont agencées par rapport au dispositif (10) côté extérieur et la section d'évacuation (16) est agencée entre l'au moins une première section d'amenée (12) et l'au moins une deuxième section d'amenée (14) et sont reliées fluidiquement à la section de prémélange (18) de sorte que l'au moins un premier fluide afflue d'une première direction dans la section de prémélange (18) et l'au moins un deuxième fluide afflue d'une deuxième direction dans la section de prémélange (18), et l'au moins une première section d'amenée (12) et l'au moins une deuxième section d'amenée (14) adoptent un angle prédéterminé l'un par rapport à l'autre, qui est configuré de sorte que le prémélange à contre-courant des fluides est généré dans la section de prémélange (18) basé sur l'écoulement turbulent et autonome.

6.  Dispositif selon l'une quelconque des revendications précédentes, dans lequel les au moins deux grandeurs d'état forment un vecteur d'état

$$v = \frac{dy}{dt} = \dot{y} \qquad (1)$$

dans lequel y représente une position et v une vitesse, et la première et la deuxième phase du filtre sont déterminées par les équations :

$$\hat{y}_{t+1} = \hat{y}_t + \Delta T \cdot \hat{v}_t \qquad (2)$$

$$\hat{v}_{t+1} = \hat{v}_t \qquad (3)$$

$$\hat{y}_t = \hat{y}_{t-1} + \alpha \cdot (y_t - \hat{y}_{t-1}) \qquad (4)$$

$$\hat{v}_t = \hat{v}_{t-1} + \frac{\beta}{\Delta T} \cdot (y_t - \hat{y}_{t-1}) \qquad (5)$$

dans lequel les équations (2) et (3) représentent la phase prédictive et les équations (4) et (5) représentent la phase corrective, les grandeurs dans les équations représentent des valeurs estimées de la grandeur respectivement mesurée et le temps de balayage entre deux mesures successives, l'index t indique la valeur actuelle par rapport à la mesure actuelle, l'index t-1 indique la valeur par rapport à respectivement la dernière mesure, l'index t+1 indique la valeur par rapport à respectivement la prochaine mesure, et les propriétés du filtre sont réglables par le biais d'un paramètre $\alpha$ et d'un paramètre $\beta$.

**7.** Procédé de prémélange de fluides en liquide de dialyse pour un traitement du sang extracorporel, avec les étapes de :

amenée d'au moins un premier fluide à partir d'au moins un premier réservoir de fluide par le biais d'au moins une première section d'amenée (12) ;
amenée d'au moins un deuxième fluide à partir d'au moins un deuxième réservoir de fluide par le biais d'au moins une deuxième section d'amenée (14) ;
prémélange des fluides amenés par le biais de la section d'amenée à contre-courant en un mélange de fluide dans une section de prémélange (18) ;
évacuation du mélange de fluide prémélangé dans la section de prémélange en tant que fluide de dialyse par le biais d'au moins une section d'évacuation (16) ; et
réalisation d'une mesure de conductibilité le long de la section d'évacuation (16) pour mesurer la conductibilité du mélange de fluide prémélangé, **caractérisé en ce que**
lors de la mesure de conductibilité, la conductibilité est mesurée au moyen d'un filtre numérique paramétrable sur la base de deux grandeurs d'état du processus de prémélange biphasé avec une première phase prédictive et une deuxième phase corrective.

**8.** Procédé selon la revendication 7, dans lequel
une des grandeurs d'état peut être déduite de respectivement l'autre,
dans la première phase prédictive, un prochain état de système est déterminé à l'aide de valeurs mesurées, et
dans la deuxième phase corrective, la détermination précédente est corrigée à l'aide de valeurs historiques, et
dans lequel les au moins deux grandeurs d'état forment un vecteur d'état

$$v = \frac{dy}{dt} = \dot{y} \tag{1}$$

dans lequel y représente une position et v une vitesse, et la première et la deuxième phase du filtre sont déterminées par les équations :

$$\hat{y}_{t+1} = \hat{y}_t + \Delta T \cdot \hat{v}_t \tag{2}$$

$$\hat{v}_{t+1} = \hat{v}_t \tag{3}$$

$$\hat{y}_t = \hat{y}_{t-1} + \alpha \cdot (y_t - \hat{y}_{t-1}) \tag{4}$$

$$\hat{v}_t = \hat{v}_{t-1} + \frac{\beta}{\Delta T} \cdot (y_t - \hat{y}_{t-1}) \tag{5}$$

dans lequel les équations (2) et (3) représentent la phase prédictive et les équations (4) et (5) représentent la phase corrective, les grandeurs dans les équations représentent des valeurs estimées de la grandeur respectivement mesurée et le temps de balayage entre deux mesures successives, l'index t indique la valeur actuelle par rapport à la mesure actuelle, l'index t-1 indique la valeur par rapport à respectivement la dernière mesure, l'index t+1 indique la valeur par rapport à respectivement la prochaine mesure, et les propriétés du filtre sont réglables par le biais d'un paramètre $\alpha$ et d'un paramètre $\beta$.

**FIG. 1**

ZU LF-MESSSONDE(N)

VORLAUFBEHÄLTER
(OSMOSEWASSER)

KONZENTRAT
(BICARBONAT)

**FIG. 2**

**FIG. 3**

**FIG. 4**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1491222 A1
- DE 102014015858 A1
- US 20140107835 A1